# EUROPEAN PATENT APPLICATION

(11) **EP 4 782 428 A1**
(43) Date of publication of application: **29.07.2026**
(21) Application number: 25214078.5
(22) Date of filing: 06.11.2025
(51) Int. Cl.: C05D 7/00, C05F 3/00, C05F 7/00, C05F 11/08, C05F 17/10, C05F 17/20, C05F 17/40, C05F 17/70, C05F 17/80, B01D 53/62, C12M 1/107, C12P 7/08

(54) **A PROCESS FOR CAPTURING CARBON DIOXIDE USING BIO-DIGESTER SLURRY LEADING TO MANURE QUALITY IMPROVEMENT**

(30) Priority: 07.11.2024 IN 202421085331
(71) Applicant: Indian Oil Corporation Limited, Mumbai, Maharashtra 400 051 (IN)
(72) Inventor: SAHOO, Prakash Chandra, Faridabad-12100 Haryana (IN); KUMAR, Manoj, Faridabad-12100 Haryana (IN); GUPTA, Ravi Prakash, Faridabad-12100 Haryana (IN); BHATTACHARYYA, Debasis, Faridabad-12100 Haryana (IN); RAMAKUMAR, Sankara Sri Venkata, Faridabad-12100 Haryana (IN)
(74) Representative: Sögüt, Onur Ömer

(57) **Abstract**

The present disclosure relates to a process for capturing CO₂ using bio-digester slurry, along with a method to improve the quality of fermented organic manure (FOM) and liquid fermented organic liquor (LFOM). The present disclosure describes the use of lignin functionalizing microbes (LFM) and H₂S oxidizing microbes (SOD) to enhance the quality of the FOM and LFOM and improve the CO₂ capture ability of the slurry. The CO₂ and /or H₂S captured manure increases their nutrient value for use in agriculture. The CO₂ absorption process not only aids in reducing the amount of greenhouse gases released into the environment, but also improves the quality of FOM and LFOM. The integration of these processes results in a sustainable and efficient approach for capturing CO₂ while improving the quality of organic waste material for agricultural use.

## Description

### FIELD OF THE INVENTION

The present disclosure relates to a process for capturing carbon dioxide using bio-digestor slurry leading to improvement in manure quality. More particularly, the present disclosure relates to a process for capturing carbon dioxide using lignin de-polymerizing microbes and H₂S oxidizing microbes. The process of the present disclosure not only enhances the quality of the fermented organic manure (FOM) and liquid fermented organic liquor (LFOM) but also helpful in reducing the emission of greenhouse gases into the environment.

### BACKGROUND OF THE INVENTION

Compressed biogas (CBG) is a renewable energy source produced from organic waste materials such as agriculture residue, food waste, and municipal solid waste. While the production of CBG is considered to be an environmentally friendly alternative to fossil fuels, the process of generating CBG also produces carbon dioxide (CO₂) emissions. The amount of CO₂ generated per ton of compressed biogas (CBG) produced can vary depending on the specific conditions of the CBG plant. However, on average, the production of one ton of CBG can result in the generation of approximately 1.5 to 2.5 tons of carbon dioxide (CO₂) emissions. This is because the process of producing CBG involves the breakdown of organic waste materials through anaerobic digestion, which results in the release of methane gas and other greenhouse gases such as CO₂ as well as H₂S.

The purification of biogas typically involves several stages, including pre-treatment, desulfurization, and CO₂ removal. During the CO₂ removal stage, the biogas is passed through a pressure swing adsorption (PSA) system/amine scrubber or a membrane separation unit to selectively capture the CO₂ molecules and the captured CO₂/H₂S is then vented.

There are several methods being explored for the capture and utilization of CO₂. Most of the methods require significant amounts of energy, which can negate the benefits of using it in the first place. Furthermore, some methods of CO₂ utilization, such as carbon capture and storage, have raised concerns about the safety and long-term storage of captured CO₂. These limitations highlight the need for continued research and development in this area to ensure that CO₂ utilization can be a viable solution to reducing carbon emissions.

The present disclosure overcomes the aforesaid limitations and provides a potential method of CO₂ utilization involving use of bio-digester slurry to capture and store CO₂ and simultaneously improve the quality of fermented organic manure (FOM) and liquid fermented organic liquor (LFOM).

Bio-digester slurry is a mixture of organic matter, ammonia and water that is produced during the anaerobic digestion of organic waste. This slurry is rich in organic compounds, making it a valuable resource for various agricultural and industrial applications. Recently, there has been growing interest in using bio-digester slurry as a medium for capturing and storing carbon dioxide (CO₂), a potent greenhouse gas that is a major contributor to climate change.

Several prior arts exist that relate to the use of bio-digester slurry for CO₂ absorption. He et.al describes a process for CO₂ absorption using biogas slurry. In said process, raw biogas slurry (RBS) may be a renewable CO₂ solvent to capture CO₂ and fix CO₂ into the crops/plants by forming organic carbon through carbon concentration mechanism (CCM). Yan et.al describes CO₂ absorption by using a low-cost solvent: biogas slurry produced by anaerobic digestion of biomass. In said process, both capacity of CO₂ absorption by using biogas slurry and the phytotoxicity of the formed CO₂-rich biogas slurry on seeds breeding was investigated. Shivali, 2021 describes an adsorption-based hydrogen sulfide removal reactor using char and spent slurry of biodigester. In said process, biogas digested slurry for biogas desulphurization is used. The said process describes that after treatment, digested slurry gets enriched about two times with sulfur S (%) than initial sulfur S (%) present in the untreated slurry. Also, the P (%) and N (%) showed a visible increase after H₂S treatment.

US2023122678A1 discloses a process for preparation of fertilizer, soil additive, soil adjuvant, soil stabilizer, plant bio-stimulant, and/or mushroom growth enhancer from inorganic and organic C1 substrates by introducing these substrates in a culture medium comprising microorganisms and in presence of electron donor. Substrate includes lignocellulosic biomass and can be in liquid form. The microorganisms includes lignin de-polymerizing microbes like *Rhodococcus jostii, Pseudomonas putida, Streptomyces sp., Comamonas sp., Nocardia sp. Gordonia sp., Bacillus sp., Cupriavidus sp, Mycobacterium sp., Rhodococcus erythropolis, Burkholderia sp., Arthrobacter sp., Thiobacillus sp., Thiomicrospira sp., Beggiatoa sp., Desulfocapsa sp.* and sulphur-oxidizers in general. Electron donors can be hydrogen sulphide.

WO2017165244A1 discloses a process for capture and conversion of an inorganic and/or organic molecule containing only one carbon atom, into organic molecules containing two or more carbon atoms through anabolic biosynthesis in presence of microorganisms and electron donor. Organic molecules can be a substrate including lignocellulosic biomass and can be in liquid form.

However, the aforesaid prior arts have several limitations like very low CO₂ loading using bio-digester slurry due to low N content moieties in the slurry. Incorporating the utilization of H₂S along with CO₂ may be technically challenging and require specialized expertise, equipment, and resources, which could increase the cost and complexity of the process. Further, the existing arts does not show any innovative methods for utilizing captured CO₂ and H₂S to improve the quality of FOM and LFOM. In addition, existing methods are not efficient in functionalizing de-polymerized lignin with nitrogen, which could have a significant impact on the improvement of CO₂ absorption and therefore limit its potential commercial value. None of the cited prior art provides a simple and cost-effective process for capturing carbon dioxide using bio-digestor slurry leading to improvement in manure quality.

In order to overcome the aforesaid drawbacks of the conventional processes, the present disclosure provides a simple and cost-effective method for capturing carbon dioxide using bio-digestor slurry leading to improvement in manure quality. The present disclosure describes the use of lignin de-polymerizing microbes, H₂S oxidizing microbes to enhance the quality of the FOM and LFOM and improves the CO₂ capture ability of the slurry. The CO₂ and/or H₂S captured manure increases their nutrient value for use in agriculture. Accordingly, the CO₂ absorption process of the present disclosure not only aids in reducing the amount of greenhouse gases released into the environment, but also improves the quality of FOM and LFOM. In view of above, the process of the present disclosure provides a sustainable and efficient process for capturing CO₂ while improving the quality of organic waste material for agricultural use.

The primary object of the present disclosure is to develop an improved process for capturing carbon dioxide using bio-digestor slurry leading to improvement in manure quality.

Another object of the present disclosure is to provide an improved microbial process for capturing carbon dioxide using bio-digestor slurry thus reducing greenhouse gas emissions.

Still another objective of the present invention is to provide an efficient method for capturing carbon dioxide along with management of organic waste thus providing improved quality of manure (FOM and LFOM) for its use in agriculture.

The process of the present disclosure is advantageous as it provides a process for capturing CO₂ using bio-digester slurry. This overcomes the limitations of previously known methods by utilizing a renewable and abundant resource for CO₂ capture. Furthermore, the process of the present disclosure addresses the need for improving the quality of fermented organic manure (FOM) and liquid fermented organic liquor (LFOM). In the process of the present disclosure, it is achieved by incorporating lignin de-polymerizing microbes and H₂S oxidizing microbes into the process. These microbes enhance the quality of FOM and LFOM, making them more beneficial for agricultural use. In addition, the invention enhances the CO₂ capture ability of the slurry, resulting in more efficient and effective CO₂ absorption. This is achieved by optimizing the composition and conditions of the bio-digester slurry, leading to increased CO₂ capture rates. Importantly, the captured CO₂ and/or H₂S in the manure significantly increases its nutrient value, making it a valuable resource for agriculture. This improves the sustainability of the process by utilizing waste materials to enhance soil fertility and crop production. The integration of these processes not only addresses the environmental concern of reducing greenhouse gas emissions but also improves the overall quality of FOM and LFOM. This sustainable and efficient approach provides a comprehensive solution for capturing CO₂ while simultaneously improving the agricultural value of organic waste materials. In summary, the advancements of the present disclosure include an improved CO₂ capture process using bio-digester slurry, the introduction of lignin de-polymerizing and H₂S oxidizing microbes to enhance the quality of FOM and LFOM, improved CO₂ capture efficiency, and increased nutrient value of the captured manure for agricultural use. These advancements collectively result in a more sustainable and efficient approach to CO₂ capture and waste management.

The method of the present disclosure addresses several problems related to CO₂ capture and the quality of organic waste materials. It includes the problem of limited CO₂ capture methods: The existing methods for capturing CO₂ often rely on expensive and energy-intensive processes whereas the method of present disclosure utilizes bio-digester slurry, a renewable and easily accessible resource, to capture CO₂ efficiently. The method of present disclosure utilizes renewable and readily available resources for efficient CO₂ capture. Further, the method of the present disclosure addresses the problem of poor quality of fermented organic manure and liquid fermented organic liquor: The existing method does not adequately address the issue of improving the quality of FOM and LFOM, which are essential for agricultural use. The process of the present disclosure overcomes this problem by incorporating lignin functionalizing microbes and H₂S oxidizing microbes into the process, thus enhancing the quality of FOM and LFOM. The process of the present disclosure improves the CO₂ capture ability of the bio-digester slurry by introducing lignin functionalizing microbes and H₂S oxidizing microbes. These microorganisms optimize the composition and conditions of the slurry, resulting in increased CO₂ capture rates and improved efficiency. These microorganisms enhance the quality of FOM and LFOM, making them more suitable for agricultural use.

Another problem addressed by the present disclosure is the low nutrient value of organic waste. The captured CO₂ and/or H₂S in the manure increases its nutrient value, making it more valuable for agricultural purposes. This addresses the problem of low nutrient content in organic waste materials and provides a sustainable solution for enhancing soil fertility and crop production. Yet another problem solved by the present disclosure is environmental impact of greenhouse gas emissions: The CO₂ absorption process of the present disclosure helps to reduce the release of greenhouse gases into the environment. By capturing CO₂ and improving the quality of FOM and LFOM, the method of the present disclosure provides an environmentally beneficial solution to mitigate the impact of greenhouse gas emission.

The present disclosure provides a method for capturing CO₂ using bio-digester slurry, which overcomes the limitations of prior art methods. This innovative approach utilizes the slurry as a medium for CO₂ capture, providing a more efficient and sustainable solution. In the process of the present disclosure, the maximum CO₂ loading occurs only at desired contact time between liquid and gas. The method of the present disclosure addresses the improvement of FOM and LFOM, crucial components of organic waste materials. By employing lignin de-polymerizing microbes and H₂S oxidizing microbes, the quality of FOM and LFOM is significantly enhanced, resulting in higher nutrient value and improved agricultural benefits. The method of the present disclosure not only focuses on the quality enhancement of organic waste materials but also improves the CO₂ capture ability of the slurry. By utilizing specific microbes as described in the method of the present disclosure, the process optimizes the CO₂ absorption process, leading to more efficient and effective CO₂ capture. The captured CO₂ and/or H₂S within the manure contribute to increasing its nutrient value. This ensures that the organic waste material obtained from the process of the present disclosure is enriched with valuable nutrients, making it highly beneficial for agricultural use.

The CO₂ absorption process implemented in the process of the present disclosure aids in reducing the release of greenhouse gases into the environment. By capturing CO₂, the process of the present disclosure provides an environmentally friendly solution that contributes to mitigating climate change and minimizing the ecological impact associated with greenhouse gas emissions.

The process of the present disclosure comprises integration of CO₂ capture, quality enhancement of FOM and LFOM, and nutrient enrichment. By combining these processes, the process of the present disclosure establishes a sustainable and efficient approach that maximizes the value derived from organic waste materials while minimizing waste generation.

In the process of the present disclosure, appropriate ratio of LFM and SOD are used leading to improvement in the inorganic sulfur content and C/N ratio in the FOM and LFOM.

### SUMMARY OF INVENTION

In an embodiment of the present disclosure, there is provided a process for capturing carbon dioxide using bio-digestor slurry leading to improvement in manure quality, the process comprises:
- feeding bio-digestor slurry obtained after biogas production into a bio-digestor slurry unit (3) of a reactor;
- introducing lignin functionalizing microbes (LFM) in a desired ratio into the bio-digestor slurry unit (3) and mixing the slurry for 1-2 hours;
- adding H₂S-oxidising microbes (SOD) in a desired ratio into the bio-digestor slurry unit (3);
- sparging an off-gas composition comprising carbon dioxide (CO₂), hydrogen sulfide (H₂S) and trace amount of nitrogen into the bio-digestor slurry unit (3) using a micro bubbler to obtain a treated bio-digestor slurry;
- transferring the treated bio-digestor slurry from bio-digestor slurry unit (3) to a solid-liquid separation system unit (5) for separating fermentable organic matter (FOM) and liquid fermented organic manure (LFOM),
wherein the off-gas composition has a liquid to gas (L/G) ratio of 15.3 to 15.6 liter/Nm³ and is sparged for a duration of 10-15 seconds.

In an embodiment of the present disclosure, there is provided a process for capturing carbon dioxide using bio-digestor slurry as described herein, wherein the LFM are added in a ratio of 0.1 ml per 10% lignin content and has CFU of 10¹¹ per ton of bio-digester slurry.

In an embodiment of the present disclosure, there is provided a process for capturing carbon dioxide using bio-digestor slurry as described herein, wherein the H₂S-oxidising microbes (SOD) are added in a ratio of 100 ml of SOD and has CFU of 10¹¹ per ton of bio-digester slurry.

In an embodiment of the present disclosure, there is provided a process for capturing carbon dioxide using bio-digestor slurry as described herein, wherein the off-gas composition is added at a flow rate of 10 m³/h for every 0.1% (w/w) increase in N content within the bio-digester slurry.

In an embodiment of the present disclosure, there is provided a process for capturing carbon dioxide using bio-digestor slurry as described herein, wherein the LFM is selected from the group consisting of *Pseudomonas putida MTCC 5869, Bacillus subtilis MTCC 5851, Bacillus sp. MTCC 5870, Pseudomonas putida MTCC 5871* or a combination thereof.

In an embodiment of the present disclosure, there is provided a process for capturing carbon dioxide using bio-digestor slurry as described herein, wherein the SOD is selected from the group consisting of *Thiobacillus sp. MTCC 25280, Thiobacillus sp. MTCC 25281, Lactobacillus sp. MTCC 25282, Mucor sp. MTCC 25283* or a combination thereof.

In an embodiment of the present disclosure, there is provided a process for capturing carbon dioxide using bio-digestor slurry as described herein, wherein the bio-digestor slurry is prepared from feedstocks selected from the group consisting of cattle dung, kitchen waste, organic fraction of municipal solid waste, paddy straw, and press mud.

In an embodiment of the present disclosure, there is provided a process for capturing carbon dioxide using bio-digestor slurry as described herein, wherein the CO₂ content in the off-gas composition ranges from 80%-99%, and wherein the H₂S content in the off-gas composition ranges from 50-5000 parts per million (ppm), and wherein the off-gas composition has a moisture content of less than 5%.

In an embodiment of the present disclosure, there is provided a process for capturing carbon dioxide using bio-digestor slurry as described herein, wherein the off-gas composition is sparged into the bio-digestor unit (3) of the bioreactor using micro-bubbler of a biogas purification unit (2).

In an embodiment of the present disclosure, there is provided a process for capturing carbon dioxide using bio-digestor slurry as described herein, wherein the reactor is a column reactor.

These and other features, aspects, and advantages of the present subject matter will be better understood with reference to the following description. This summary is provided to introduce a selection of concepts in a simplified form.

### BRIEF DESCRIPTION OF THE ACCOMPANYING DRAWINGS

The following figures form part of the present specification and are included to further illustrate aspects of the present disclosure. The disclosure may be better understood by reference to the figures in combination with the detailed description of the specific embodiments presented herein.

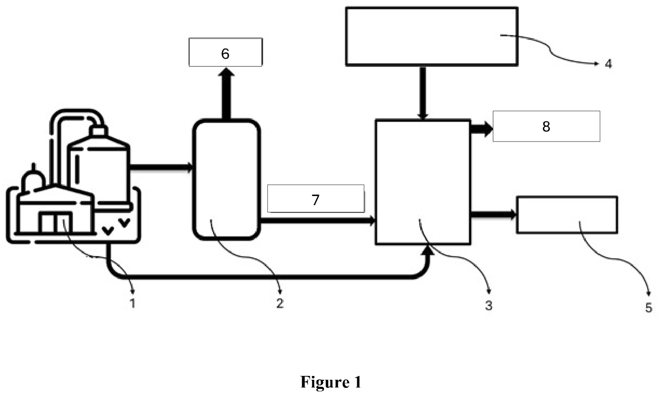

Figure 1 is a schematic representation of the process of the present disclosure.

### DETAILED DESCRIPTION OF THE INVENTION

Those skilled in the art will be aware that the present disclosure is subject to variations and modifications other than those specifically described. It is to be understood that the present disclosure includes all such variations and modifications. The disclosure also includes all such steps of the process, features of the product, referred to or indicated in this specification, individually or collectively, and any and all combinations of any or more of such steps or features.

### Definitions

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods, and materials are now described. All publications mentioned herein are incorporated herein by reference.

The present disclosure is not to be limited in scope by the specific embodiments described herein, which are intended for the purposes of exemplification only. Functionally equivalent products and methods are clearly within the scope of the disclosure, as described herein.

The present disclosure relates to a process for capturing CO₂ using bio-digester slurry, along with a method to improve the quality of fermented organic manure (FOM) and liquid fermented organic liquor (LFOM). The present disclosure comprises the use of lignin de-polymerizing microbes, H₂S oxidizing microbes to enhance the quality of the FOM and LFOM and improve the CO₂ capture ability of the slurry. The CO₂ and/or H₂S captured manure increases their nutrient value for use in agriculture. The CO₂ absorption process not only aids in reducing the amount of greenhouse gases released into the environment, but also improves the quality of FOM and LFOM. The integration of these processes results in a sustainable and efficient approach for capturing CO₂ while improving the quality of organic waste material for agricultural use.

In an aspect of the present disclosure, there is provided a process for capturing CO₂ using bio-digester slurry and simultaneously improving the quality of fermented organic manure (FOM) and liquid fermented organic liquor (LFOM) prepared from the CO₂ enriched bio-digester slurry. The process comprises the following major steps:
- Collecting CO₂ and/or H₂S gases from the outlet of a scrubber after biogas purification.
- Gathering bio-digester slurry in a container.
- Introducing lignin de-polymerizing and H₂S-oxidizing microbes in a desired ratio.
- Injecting the CO₂ and/or H₂S gases into the digester slurry at an appropriate L/G ratio for a predetermined duration.
- Transferring the treated digester slurry to a solid-liquid separation system (SLS) for separating fermentable organic matter (FOM) and LFOM.

In an embodiment, the present disclosure provides a process for capturing carbon dioxide using bio-digestor slurry leading to improvement in manure quality. The steps of the method of the present disclosure are performed in following units:

### A. Feed gas unit/ Biogas purification unit

The first step of the method of the present disclosure comprises collecting CO₂ and/or H₂S gases from the outlet of a scrubber after biogas purification. The CO₂ concentration from the outlet of biogas purification unit ranges from around 80% to 99% and the H₂S concentration from the outlet of biogas purification unit ranges from around 30-3000 ppm.

Preferably, the gases from the biogas purification unit are directly routed through any pipe to the bio-digester slurry unit.

The flow rate of the CO₂/H₂S to the bio-digester slurry depends on the N content on the bio-digester slurry.

### B. Bio-digester slurry unit

The bio-digester slurry obtained after biogas production contains water, organic materials, nutrients, microorganisms, soluble compounds, and solid residues.

The bio-digester slurry coming out of the digester after biogas production needs to be stored in a closed vessel.

In the method of the present disclosure, the biodigester slurry obtained after biogas production is fed into bio-digestor unit of a reactor.

The bio-digestor unit of the reactor has provision for adding lignin de-polymerizing microbes, H₂S oxidizing microbes.

Lignin functionalizing microbes (LFM) are added in a ratio of 800 ml of LDPM having CFU of 10¹¹ per Ton of bio-digester slurry. After adding LFM, the slurry is mixed for 2 hours.

H₂S oxidizing microbes (SOD) are added subsequently in a ratio of 100 ml of SOD having CFU of 10¹¹ per Ton of bio-digester slurry.

After addition of the LFM and SOD, the CO₂/H₂S obtained is sparged into the slurry from below.

The flow rate of CO₂/H₂S is to be decided based on N content in the bio-digester slurry.

### C. LFOM and FOM production and characterization

The FOM and LFOM to be separated by solid and liquid separator.

The FOM and LFOM to be characterized by various parameters.

In a preferred embodiment of the present disclosure, there is provided a process for capturing carbon dioxide using bio-digestor slurry leading to improvement in manure quality, the process comprises:
- feeding bio-digestor slurry obtained after biogas production into a bio-digestor slurry unit (3) of a reactor;
- introducing lignin functionalizing microbes (LFM) in a desired ratio into the bio-digestor slurry unit (3) and mixing the slurry for 1-2 hours;
- adding H₂S-oxidising microbes (SOD) in a desired ratio into the bio-digestor slurry unit (3);
- sparging an off-gas composition comprising carbon dioxide (CO₂), hydrogen sulfide (H₂S) and trace amount of nitrogen into the bio-digestor slurry unit (3) using a micro bubbler to obtain a treated bio-digestor slurry.
- transferring the treated bio-digestor slurry from bio-digestor slurry unit (3) to a solid-liquid separation system unit (5) for separating fermentable organic matter (FOM) and liquid fermented organic manure (LFOM),
wherein the off-gas composition has a liquid to gas (L/G) ratio of 15.3 to 15.6 liter/Nm³ and is sparged for a duration of 10-15 seconds.

In an embodiment of the present disclosure, the methods used for biogas purification include but not limited to water scrubbing, pressure swing adsorption (PSA), chemical absorption, and membrane separation.

The off-gas composition in the biogas purification unit after methane purification includes (a) Carbon Dioxide (CO₂), (b) Hydrogen Sulfide (H₂S), (c) Moisture, and (d) Trace Impurities like nitrogen (N₂), oxygen (O₂), trace hydrocarbons, and other volatile organic compounds (VOCs). These impurities are usually present in very low concentrations, often below 1% or even lower.

In an embodiment of the present disclosure, there is provided a process for capturing carbon dioxide using bio-digestor slurry as described herein, wherein the CO₂ content in the off-gas composition ranges from 80%-99%, and wherein the H₂S content in the off-gas composition ranges from 50-5000 parts per million (ppm), and wherein the off-gas composition has a moisture content of less than 5%.

In an embodiment of the present disclosure, the material of construction (MOC) suitable for handling CO₂ and H₂S in the context of transferring gases from a biogas purification unit to a digester slurry include Stainless Steel, High-Density Polyethylene (HOPE), Corrosion-Resistant Alloys, Fiberglass Reinforced Plastic (FRP) or a combination thereof.

The flow rate of the off-gas composition comprising carbon dioxide (CO₂) and hydrogen sulfide (H₂S) to the bio-digester slurry is based on the nitrogen (N) content present in the slurry. The flow rate is adjusted to maintain a specific N concentration in the slurry for efficient capture of CO₂.

In an embodiment of the present disclosure, there is provided a process for capturing carbon dioxide using bio-digestor slurry as described herein, wherein the off-gas composition is added at a flow rate of 10 m³/h for every 0.1% (w/w) increase in N content within the bio-digester slurry.

In an embodiment of the present disclosure, the relationship between flow rate of off-gas composition and N content within the bio-digester slurry allows for fine-tuning of the CO₂ flow rate based on the N concentration to optimize the efficiency of CO₂ capture.

In an embodiment of the present disclosure, the transfer of the anaerobic digester slurry to a separate vessel equipped with inlet and outlet provisions for CO₂ and H₂S gases.

In an embodiment of the present disclosure, there is provided a process for capturing carbon dioxide using bio-digestor slurry as described herein, wherein the bio-digestor slurry is prepared from feedstocks selected from the group consisting of cattle dung, kitchen waste, organic fraction of municipal solid waste, paddy straw, and press mud.

A variety of feedstock was used for preparing the bio-digestor slurry. It includes cattle dung obtained from Faridabad, Haryana, India, kitchen waste obtained from IOCL R&D Canteen, Faridabad, Haryana, India, organic fraction of municipal solid waste obtained from Faridabad district, Haryana, India, paddy straw obtained from Palwal district, Faridabad, Haryana and press mud obtained from Faridabad, India.

In an embodiment of the present disclosure, the reactor for the transfer of the anaerobic digester slurry to a separate vessel as described herein is preferably a column reactor, specifically chosen for its ability to facilitate improved interaction between the liquid and slurry components.

In an embodiment of the present disclosure, the material of construction (MOC) for the reactor used for the transfer of the anaerobic digester slurry to a separate vessel is selected from Stainless Steel, High-Density Polyethylene (HOPE), Corrosion-Resistant Alloys, Fiberglass Reinforced Plastic (FRP), or a combination thereof. The choice of MOC depends on factors such as the corrosive nature of the gases and liquids involved, the desired durability, and the compatibility with the specific operating conditions.

In an embodiment of the present disclosure, the column reactor design is selected to optimize the gas-liquid interaction within the slurry. This promotes efficient mass transfer and enhances the desired reactions between the CO₂ and H₂S gases and the slurry components. The column reactor provides an environment that allows longer contact time and improved gas-liquid mixing, thereby facilitating the desired conversion processes.

The Lignin functionalizing microbes (LFM) are microorganisms that have the ability to modify lignin by introducing nitrogen-based functional groups into its structure. These microbes possess specific enzymes, such as lignin peroxidases and laccases, which can break down lignin and incorporate nitrogen atoms into its chemical composition. This process is known as lignin nitrogen functionalization.

In an embodiment of the present disclosure, the LFM microbes is selected from the group consisting of *Rhodococcus jostii* RHA1, *Pseudomonas putida* KT2440, *Streptomyces viridosporus* T7A, *Novosphingobium aromaticivorans* DSM 12444, *Sphingobium* sp. SYK-6, *Comamonas testosteroni* CNB-1, *Nocardia* sp. NRRL 5646, *Gordonia* sp. strain MTCC 4818, *Bacillus subtilis* subsp. subtilis 168, *Bacillus subtilis MTCC 5851, Bacillus sp. MTCC 5870, Cupriavidus basilensis* B-*8, Mycobacterium smegmatis* MC2 155, *Thermobifida fusca* YX, *Rhodococcus erythropolis* PR4, *Bacillus halodurans* C-125, *Sinorhizobium meliloti* 1021, *Burkholderia xenovorans* LB400, *Amycolatopsis* sp. ATCC 39116, *Nocardia farcinica* IFM 10152, *Arthrobacter* sp. strain C1A, *Pseudomonas putida MTCC 5869, Pseudomonas putida MTCC 5871, Sphingomonas* sp. strain MM-*1, Streptomyces griseus* NBRC 13350.

In a preferred embodiment of the present disclosure, the LFM microbe is selected from the group consisting of *Pseudomonas putida MTCC 5869, Bacillus subtilis MTCC 5851, Bacillus sp. MTCC 5870, Pseudomonas putida MTCC 5871* or a combination thereof.

In an embodiment of the present disclosure, the media composition for cultivating *Pseudomonas putida MTCC 5871* typically includes: (a) Carbon source: 0.5-1% w/v of glucose or another suitable carbon source, (b) Nitrogen source: 0.2-0.5% w/v of ammonium sulfate or another nitrogen source, (c) Minerals: Include essential minerals such as potassium, magnesium, calcium, phosphorus, and trace elements in appropriate concentrations, (d) Buffering agent: Add a suitable buffering agent to maintain the pH of the medium around 7.0, (e) the media optionally comprises Agar : If solid media is desired, add agar at a concentration of 1.5-2.0% w/v. The medium is autoclaved at 121°C for 15-20 minutes to ensure sterility.

The lignin content in anaerobic biodigester slurry is typically in the range of 10-30% based on dry weight. The specified LFM ratio is based on the lignin content in the slurry, ensuring optimal microbial activity. This embodiment contributes to improved functionalization efficiency and overall performance of the biodigester system.

In an embodiment of the present disclosure, there is provided a process for capturing carbon dioxide using bio-digestor slurry as described herein, wherein the LFM are added in a ratio of 0.1 ml per 10% lignin content.

The LFM are added to the biodigester slurry in sufficient population to enhance lignin functionalization within the slurry. In an embodiment of the present disclosure, the Lignin Functionalizing Microbes (LFM) has a colony-forming unit (CFU) count of 10¹¹ per ton of bio-digester slurry.

In an embodiment of the present disclosure, after adding LFM to the biodigester slurry, the slurry is allowed to be mixed for 1-2 hours.

In an embodiment, the process of the present disclosure comprises adding H₂S oxidizing microbes (SOD) to the biodigester slurry.

SOD includes sulfur-oxidizing bacteria that use H₂S as an energy source for their metabolism and convert it into elemental sulfur as a byproduct. This process is important for sulfur cycling in natural environments and can have implications for industrial applications, such as bio-desulfurization in wastewater treatment or sulfur recovery in the petroleum industry.

In an embodiment of the present disclosure, the SOD microbes are selected from the group consisting of *Acidithiobacillus thiooxidans, Acidithiobacillus ferrooxidans, Lactobacillus sp. MTCC 25282, Mucor sp. MTCC 25283, Thiobacillus denitrificans, Thiobacillus thioparus, Thiobacillus novellus, Thiobacillus sp. MTCC 25280, Thiobacillus sp. MTCC 25281, Thiobacillus neapolitanus, Thiomicrospira denitrificans, Thiomicrospira crunogena, Thioalkalivibrio sulfidiphilus, Sulfurimonas denitrificans, Sulfitricurvum kujiense, Sulfurospirillum deleyianum, Sulfurospirillum multivorans, Sulfurovum lithotrophicum, Thioalkalivibrio nitratireducens, Thioalkalivibrio sulfidophilus, Thioalkalimicrobium cyclicum, Beggiatoa* spp., *Desulfocapsa sulfoexigens, Desulfococcus multivorans* or a combination thereof.

In a preferred embodiment of the present disclosure, the SOD microbes are selected from the group consisting of *Thiobacillus sp. MTCC 25280, Thiobacillus sp. MTCC 25281, Lactobacillus sp. MTCC 25282, Mucor sp. MTCC 25283* or a combination thereof.

In an embodiment of the present disclosure, the media composition for cultivating SOD typically includes the following components in specific quantities: Ammonium sulfate: 0.5 g/L, Potassium phosphate (dibasic): 0.2 g/L, Magnesium sulfate: 0.1 g/L, Iron(II) sulfate heptahydrate: 0.01 g/L, Manganese(II) sulfate: 0.01 g/L, Zinc sulfate: 0.01 g/L, Glucose: 5 g/L (as the carbon source), Distilled water: Sufficient to make up the desired volume. Further, the pH of the medium used to cultivate the SOD microbes is adjusted to around 2.0 using a suitable acid, such as sulfuric acid or hydrochloric acid. Furthermore, aeration is provided to ensure the presence of dissolved oxygen for the bacterium's sulfur-oxidizing activity.

In an embodiment of the present disclosure, there is provided a process for capturing carbon dioxide using bio-digestor slurry as described herein, wherein the H₂S-oxidising microbes (SOD) are added in a ratio of 100 ml of SOD and is having CFU of 10¹¹ per ton of bio-digester slurry.

In an embodiment of the present disclosure, the CO₂/H₂S are inserted into the bio-digestor slurry from below the reactor through a micro bubbler.

In an embodiment of the present disclosure, the off-gas composition comprising CO₂ and H₂S gases, generated from appropriate sources, are fed into the micro-bubbler system.

In the method of the present disclosure, the gas flow rate is carefully controlled to achieve optimal gas-liquid mass transfer and maintain the desired reaction conditions. The gas flow rate is adjusted to ensure a longer residence time within the reactor. By controlling the gas flow, the contact time between the gas and liquid is extended to the desired range.

In an embodiment of the present disclosure, the contact time between the gas and liquid is in the range of 10-15 seconds.

In an embodiment of the present disclosure, the off-gas composition has a liquid to gas (L/G) ratio maintained at 15.3-15.6 liter of slurry per standard cubic meter (Nm³) of feed gas/off-gas composition to ensure 30-60% CO₂ utilization using the biodigester slurry.

In an embodiment of the present disclosure, fermented organic manure (FOM) and liquid fermented organic liquor (LFOM) are separated using solid and liquid separator (SLS).

The SLS can include Centrifuges, Sedimentation Tanks, and Filters, cyclones, Membrane Separators or a combination thereof.

Finally, FOM and LFOM are characterized by various parameters before and after CO₂/H₂S utilization. In an embodiment, the produced FOM and LFOM (compost) were tested for seed germination test for toxicity evaluation of compost.

In an embodiment of the present disclosure, the seed germination test for toxicity evaluation of compost was conducted following a standardized procedure.

Representative compost samples were collected and air-dried for uniformity. The compost samples were then ground into smaller particles. Lettuce seeds were chosen as the test seeds and were placed on moistened filter paper in sterilized Petri dishes. A measured amount of compost (10% w/w) was added to the designated dishes, ensuring even distribution over the seeds. Controlled dishes with sterile water were prepared for comparison.

The dishes were covered and placed in an incubator with optimal germination conditions. Germination progress was monitored, and the number of germinated seeds was recorded. Germination percentages and rates were calculated for each compost sample and control group. Abnormal seedling growth was also observed and documented.

Data analysis was performed, comparing the germination percentages and rates between the compost samples and the control. Statistical methods were used to determine significant differences and assess compost toxicity.

The toxicity of compost samples was evaluated based on germination performance compared to the control group. Lower germination percentages or rates, along with observable abnormalities, indicated potential toxicity or inhibitory effects.

Although the subject matter has been described in considerable detail with reference to certain preferred embodiments thereof, other embodiments are possible.

### EXAMPLES

The disclosure will now be illustrated with working examples, which are intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods, the exemplary methods, devices and materials are described herein. It is to be understood that this disclosure is not limited to particular methods, and experimental conditions described, as such methods and conditions may vary.

### Example 1: CO₂/H₂S capture using cattle dung based biodigester slurry

20 L of bio-digester slurry after 21 days of fermentation was collected from cattle dung based CBG plant having the following characteristics, as mentioned in table-1 below.

**Table-1: Characteristics of Cattle dung (CD) biodigester slurry used for CO₂/H₂S capture.**

| **Cattle dung (CD) biodigester slurry** | |
|---|---|
| **Parameter** | **Range** |
| pH (10% solution) | 7.9 |
| Electrical conductivity (dS/m) | 3.61 |
| Moisture (% w/w) | 30.29 |
| Total Nitrogen (% w/w) | 0.86 |
| Total Phosphorous (% w/w) | 0.64 |
| Potassium content (% w/w) | 0.59 |
| Total N, P, K (% w/w) | 2.09 |
| Total Organic Carbon (min) % w/w | 10.3 |
| Particle size | 90.4% material passes |
| Inorganic sulfur (%w/w) | 0.2 |
| C/N ratio | 11.98 |

Synthetic feed of CBG purification plant outlet was prepared (95% CO₂ + 2% H₂S + Balance N₂).

A bioreactor (30L capacity) was prepared having provision for gas sparing from below and out of the gas was collected from the top.

The bioreactor inlet and outlet were connected with CO₂ and H₂S measurement analyzer.

The slurry (20L) was inserted into a bioreactor and 160 ml of *Pseudomonas putida MTCC 5871* was inserted into the slurry. *Pseudomonas putida MTCC 5871* was initially cultivated in Carbon source: 1% w/v of glucose or another suitable carbon source, Nitrogen source: 0.5% w/v of ammonium sulfate or another nitrogen source, Minerals: Include essential minerals such as potassium, magnesium, calcium, phosphorus, and trace elements in appropriate concentrations, Buffering agent: Add a suitable buffering agent to maintain the pH of the medium around 7.0, Agar (optional): If solid media is desired, add agar at a concentration of 12.0% w/v, Sterilization: Autoclave the medium at 121°C for 15 minutes to ensure sterility. The initial CFU of the microbes was maintained as 2x10¹¹.

After adding *Pseudomonas putida MTCC 5871* to biodigester, the slurry to be mixed for 2 hours.

After 2 hours, 20 ml of *Thiobacillus sp. MTCC 25280* having CFU of 5.2x10¹¹ was then bio-digester slurry.

*Thiobacillus sp. MTCC 25280* was initially cultivated in a media having composition: Ammonium sulfate: 0.5 g/L, Potassium phosphate (dibasic): 0.2 g/L, Magnesium sulfate: 0.1 g/L, Iron(II) sulfate heptahydrate: 0.01 g/L, Manganese(II) sulfate: 0.01 g/L, Zinc sulfate: 0.01 g/L, Glucose: 5 g/L (as the carbon source), Distilled water: Sufficient to make up the desired volume, The pH of the medium is adjusted to around 2.0 using a suitable acid, such as sulfuric acid or hydrochloric acid.

Initially the gas flow rate was maintained as 20 liter of slurry per standard cubic meter (Nm³) of feed gas for 6 hours.

The concentration of inlet CO₂ was maintained as 95% CO₂ + 2% H₂S + Balance N₂.

The volume of gas in the outlet was measured using a portable analyzer based on which the mass balance was calculated. The results are shown in table 2 below.

**Table-2: The CO₂/H₂S conversion using CD based biodigester slurry at various treatment conditions.**

| **Material** | **Inlet feed CO₂/H₂S** | **Outlet feed CO₂/H₂S** | **Quantity of CO₂/H₂S conversion/m3 of feed gas** |
|---|---|---|---|
| Cattle dung (CD) biodigester slurry (Control) | Inlet CO₂= 1.88 kg | Outlet CO₂=1.65 Kg | CO₂=0.23 kg |
| | Inlet H₂S = 0.0279 Kg | Outlet H₂S= 0.0273 Kg | H₂S= 0.0006 kg |
| Cattle dung (CD) biodigester slurry-Treated with LFM | Inlet CO₂= 1.88 kg | Outlet CO₂= 1.24 Kg | CO₂=0.64 kg |
| | Inlet H₂S = 0.0279 Kg | Outlet H₂S= 0.0220 Kg | H₂S= 0.0059kg |
| Cattle dung (CD) biodigester slurry-SOD | Inlet CO₂= 1.88 kg | Outlet CO₂= 1.61 Kg | CO₂=0.26 kg |
| | Inlet H₂S = 0.0279 Kg | Outlet H₂S= 0.0131 Kg | H₂S= 0.0148 kg |
| Cattle dung (CD) biodigester slurry-Treated by LFM and SOD | Inlet CO₂= 1.88 kg | Outlet CO₂= 0.69 Kg | CO₂=1.19 kg |
| | Inlet H₂S = 0.0279 Kg | Outlet H₂S= 0.0002 Kg | H₂S= 0.0277 kg |

### Example 2: Effect of feed gas/off-gas composition and biodigester slurry contact time on CO₂/H₂S utilization

In the method of the present disclosure, the contact time between Cattle dung (CD) biodigester slurry treated by LFM and SOD and gas has been optimized.

In order to calculate the optimized contact time for maximum CO₂ absorption, the experiments were conducted where all the conditions remain constant as per Example 1 of the present disclosure.

The column height of the bioreactor was adjusted to vary the Gas and liquid contact time. The results obtained are shown in table 3 below.

Effect of feed/off gas and biodigester slurry contact time on CO₂/H₂S utilization.

| **Gas and Liquid contact time (S)** | **CO₂ conversion (in Kg) by the digester** | **H₂S Conversion (in kg) by the digester** |
|---|---|---|
| **2** | 0.52 | 0.005 |
| **5** | 0.72 | 0.012 |
| **10** | 1.19 | 0.027 |
| **20** | 1.20 | 0.027 |
| **50** | 1.20 | 0.028 |

### Example 3: CO₂ capture potential of bio-digester slurry of various feed stocks

In order to determine the CO₂ capture potential of biodigester slurry derived from different feedstocks, all parameters were kept constant based on Example 1, while the composition of the biodigester slurry from various feedstocks was altered. The results are shown in table 4 below.

**Table 4: CO₂ capture potential of bio-digester slurry of various feed stocks**

| **Biodigester slurry** | **CO₂ capture potential (Kg)/ton of slurry** |
|---|---|
| Cattle dung (CD) | 5.93 |
| Kitchen waste (KW) | 6.12 |
| Organic fraction of municipal solid waste (OF-MSW) | 6.23 |
| Press mud (PM) | 5.82 |
| Paddy straw (PS) | 6.33 |

### Example 4: Comparison of various characteristics of biodigester slurry before and after CO₂/H₂S capture at optimized condition.

The bio-digester slurry as per Example 1 was collected after CO₂/H₂S capture and various parameters were analyzed. The characteristics of biodigester slurry were given in Table-5 below.

**Table.5: Characterization of biodigester slurry before and after CO₂/H₂S utilization**

| | **Cattle dung (CD) biodigester slurry** | **Cattle dung (CD) biodigester slurry after treatment** |
|---|---|---|
| **Parameter** | **Range** | **Range** |
| pH (10% solution) | 7.9 | 7.5 |
| Electrical conductivity (dS/m) | 3.61 | 2.47 |
| Moisture (% w/w) | 30.29 | 31.29 |
| Total Nitrogen (% w/w) | 0.86 | 2.09 |
| Total Phosphorous (% w/w) | 0.64 | 1.67 |
| Potassium content (% w/w) | 0.59 | 0.93 |
| Total N, P, K (% w/w) | 2.09 | 4.69 |
| Total Organic Carbon (min) % w/w | 10.3 | 27.49 |
| Particle size | 90.4% material passes | 97.5% material passes |
| Inorganic sulfur (%w/w) | 0.2 | 1.9 |
| C/N ratio | 11.98 | 13.15 |

### Example 5: Characterization of FOM and LFOM after CO₂/H₂S utilization using Cattle dung based biodigester slurry.

The bio-digester slurry as per Example 1 was collected after CO₂/H₂S capture and subjected to solid and liquid separation.

The solid fermented organic manure (FOM) and liquid fermented organic manure (LFOM) were collected and subjected to analysis. The various parameters of FOM and LFOM were given in table 6 below.

The control sample of FOM and LFOM were also calculated. For the control samples the biodigester slurry was not treated by CO₂/H₂S.

**Table 6: Characterization of FOM and LFOM after CO₂/H₂S vis-à-vis control (untreated).**

| | **FOM (Control)** | **FOM (Treated)** | **LFOM (Control)** | **LFOM (Treated)** |
|---|---|---|---|---|
| **Parameter** | **Range** | **Range** | **Range** | **Range** |
| pH (10% solution) | 7.9 | 7.5 | 7.8 | 7.6 |
| Electrical conductivity (dS/m) | 3.61 | 2.47 | 3.62 | 2.45 |
| Moisture (% w/w) | 30.29 | 31.29 | 90.87 | 96.12 |
| Total Nitrogen (% w/w) | 0.86 | 2.39 | 0.85 | 2.34 |
| Total Phosphorous (% w/w) | 0.64 | 1.67 | 0.63 | 1.66 |
| Potassium content (% w/w) | 0.59 | 0.93 | 0.6 | 0.92 |
| Total N, P, K (% w/w) | 2.09 | 4.99 | 2.08 | 4.92 |
| Total Organic Carbon (min) % w/w | 10.76 | 22.49 | 10.84 | 22.39 |
| Particle size | 90.4% material passes | 97.5% material passes | - | - |
| C/N ratio | 12.51 | 9.41 | 12.75 | 9.57 |
| Inorganic sulfur | 0.1 | 1.6 | 0.1 | 1.4 |

### Example 6: Characterization of FOM and LFOM after CO₂/H₂S utilization using Kitchen waste (KW) based biodigester slurry.

The bio-digester slurry (using KW biodigester slurry) as per Example 1 was collected after CO₂/H₂S capture and subjected to solid and liquid separation.

The solid fermented organic manure (FOM) and liquid fermented organic manure (LFOM) were collected and subjected to analysis. The various parameters of FOM and LFOM were given in table 7 below.

The control sample of FOM and LFOM were also calculated. For the control samples the biodigester slurry was not treated by CO₂/H₂S.

**Table.7: Characterization of FOM and LFOM after CO₂/H₂S vis-à-vis control (untreated).**

| | **FOM (Control)** | **FOM (Treated)** | **LFOM (Control)** | **LFOM (Treated)** |
|---|---|---|---|---|
| **Parameter** | **Range** | **Range** | **Range** | **Range** |
| **pH** | 7.22 | 7.08 | 7.6 | 7.2 |
| **EC (µS/cm)** | 0.54 | 1.01 | 3.46 | 1.93 |
| **Total "N"** | 0.76 | 2.56 | 0.79 | 2.15 |
| **Total "P"** | 0.62 | 1.19 | 0.54 | 1.62 |
| **Total "K"** | 0.53 | 1.57 | 0.57 | 0.92 |
| **Total N, P, K (% w/w)** | 1.91 | 5.32 | 1.9 | 4.69 |
| **C: N Ratio** | 13.50 | 9.05 | 14.08 | 11.25 |
| **TOC (total organic carbon)** | 10.26 | 23.16 | 11.12 | 24.18 |
| Inorganic sulfur (%w/w) | 0.2 | 1.9 | 0.12 | 2.1 |

### Example 7: Characterization of FOM and LFOM after CO₂/H₂S utilization using municipal solid waste (MSW) based biodigester slurry.

The bio-digester slurry (using MSW biodigester slurry) as per Example 1 was collected after CO₂/H₂S capture and subjected to solid and liquid separation.

The resulting solid fractions, known as fermented organic manure (FOM), and the liquid fraction, known as liquid, fermented organic manure (LFOM), were collected for analysis. Below table 8 provides detailed the various parameters of both FOM and LFOM.

**Table 8: Characterization of FOM and LFOM after CO2/H2S vis-à-vis control (untreated).**

| | **FOM (Control)** | **FOM (Treated)** | **LFOM (Control)** | **LFOM (Treated)** |
|---|---|---|---|---|
| **Parameter** | **Range** | **Range** | **Range** | **Range** |
| **pH** | 7.28 | 7.06 | 7.4 | 7.3 |
| **EC (µS/cm)** | 0.56 | 1.04 | 3.29 | 2.43 |
| **Total "N"** | 0.62 | 2.61 | 0.71 | 2.29 |
| **Total "P"** | 0.63 | 1.22 | 0.58 | 1.77 |
| **Total "K"** | 0.55 | 1.61 | 0.63 | 0.99 |
| **Total N, P, K (% w/w)** | 1.8 | 5.44 | 1.92 | 5.05 |
| **C: N Ratio** | 16.31 | 9.42 | 14.41 | 11.19 |
| **TOC (total organic carbon)** | 10.11 | 24.58 | 10.23 | 25.63 |
| Inorganic sulfur (%w/w) | 0.1 | 1.8 | 0.15 | 1.7 |

### Example 8: Characterization of FOM and LFOM after CO₂/H₂S utilization using paddy straw based biodigester slurry.

The bio-digester slurry (using paddy straw biodigester slurry) as per Example 1 was collected after CO₂/H₂S capture and subjected to solid and liquid separation.

The resulting solid fraction, known as fermented organic manure (FOM), and the liquid fraction, known as liquid, fermented organic manure (LFOM), were collected for analysis. Table 9 below provides details of the various parameters of both FOM and LFOM.

**Table.9: Characterization of FOM and LFOM after CO₂/H₂S vis-à-vis control (untreated).**

| | **FOM (Control)** | **FOM (Treated)** | **LFOM (Control)** | **LFOM (Treated)** |
|---|---|---|---|---|
| **Parameter** | **Range** | **Range** | **Range** | **Range** |
| **pH** | 7.09 | 7.02 | 7.4 | 7.2 |
| **EC (µS/cm)** | 0.49 | 1.02 | 3.54 | 2.16 |
| **Total "N"** | 0.95 | 3.06 | 0.78 | 2.19 |
| **Total "P"** | 0.58 | 1.28 | 0.68 | 1.64 |
| **Total "K"** | 0.62 | 1.68 | 0.63 | 0.94 |
| **Total N, P, K (% w/w)** | 2.15 | 6.02 | 2.09 | 4.77 |
| **C: N Ratio** | 12.93 | 9.23 | 15.86 | 12.03 |
| **TOC (total organic carbon)** | 12.28 | 28.23 | 12.37 | 26.34 |
| **Inorganic sulfur (%w/w)** | 0.2 | 1.7 | 0.17 | 2.01 |

### Example 9: Characterization of FOM and LFOM after CO₂/H₂S utilization using press mud based biodigester slurry.

The bio-digester slurry (using press mud biodigester slurry) as per Example 1 was collected after CO₂/H₂S capture and subjected to solid and liquid separation.

The solid fermented organic manure (FOM) and liquid fermented organic manure (LFOM) were collected and subjected to analysis. The various parameters of FOM and LFOM were given in table 10 below.

The control sample of FOM and LFOM were also calculated. For the control samples the biodigester slurry was not treated by CO₂/H₂S.

**Table 10: Characterization of FOM and LFOM after CO₂/H₂S vis-à-vis control (untreated).**

| | **FOM (Control)** | **FOM (Treated)** | **LFOM (Control)** | **LFOM (Treated)** |
|---|---|---|---|---|
| **Parameter** | **Range** | **Range** | **Range** | **Range** |
| **pH** | 7.18 | 6.97 | 7.6 | 7.5 |
| **EC (µS/cm)** | 0.51 | 0.98 | 3.54 | 2.22 |
| **Total "N"** | 0.89 | 2.96 | 0.78 | 2.09 |
| **Total "P"** | 0.59 | 1.12 | 0.62 | 1.72 |
| **Total "K"** | 0.57 | 1.93 | 0.62 | 0.95 |
| **Total N, P, K (% w/w)** | 2.05 | 6.01 | 2.02 | 4.76 |
| **C: N Ratio** | 13.39 | 9.20 | 15.65 | 12.04 |
| **TOC (total organic carbon)** | 11.92 | 27.22 | 12.21 | 25.16 |
| Inorganic sulfur (%w/w) | 0.14 | 2.3 | 0.12 | 1.9 |

### Example 10: Seed germination test for toxicity evaluation of treated FOM and LFOM.

The treated FOM and LFOM from Example 1 were subjected to Seed germination test. The test was conducted using green gram seeds obtained from Faridabad district, Haryana, India. Different FOM and LFOM samples were used for comparisons.

The test was conducted in a plant growth chamber with day/night temperatures of 24°C/17°C and 13 hours of light per day.

The moisture content of the control soil and compost-soil mix was maintained at two thirds of the water retention capacity.

Seeds were initially watered from the bottom until established, and then watered from the surface.

The experiment was carried out for 7 days after 50 percent of the control seeds had germinated.

Measurements were taken for percent germination, length of seedlings, and fresh weight of above-ground material. The details are given in Table 10 below.

**Table 10: Effect of FOM and LFOM on growth of green gram**

| **Manure** | **Plant Length (cm)** | **Roots Dry Weight (g pot-1)** | **Shoots Dry Weight (g pot-1)** |
|---|---|---|---|
| **Control** | 4.3 | 1.2 | 3.2 |
| **FOM (Untreated)** | 6.2 | 1.9 | 4.1 |
| **FOM (Treated)** | 8.3 | 3.8 | 6.6 |
| **LFOM (Untreated)** | 5.8 | 2.1 | 4.3 |
| **LFOM (Treated)** | 8.9 | 3.9 | 5.9 |

### Reference Signs:

- 1: biogas plant
- 2: biogas purification unit
- 3: bio-digester slurry
- 4: Lignin functionalizing microbes (LFM), H₂S oxidizing microbes (SOD)
- 5: high-quality FOM and LFOM
- 6: bio-CNG
- 7: CO₂/H₂S rich gas
- 8: CO₂/H₂S lean gas

## Claims

1. A process for capturing carbon dioxide using a bio-digestor slurry leading to improvement in manure quality, the process comprises:
- feeding the bio-digestor slurry obtained after biogas production into a bio-digestor slurry unit (3) of a reactor;
- introducing lignin functionalizing microbes (LFM) into the bio-digestor slurry unit (3) and mixing the slurry for 1-2 hours;
- adding H₂S-oxidising microbes (SOD) into the bio-digestor slurry unit (3);
- sparging an off-gas composition comprising carbon dioxide (CO₂), hydrogen sulfide (H₂S) and trace amount of nitrogen into the bio-digestor slurry unit (3) using a micro bubbler to obtain a treated bio-digestor slurry;
- transferring the treated bio-digestor slurry from the bio-digestor slurry unit (3) to a solid-liquid separation system unit (5) for separating a fermentable organic matter (FOM) and a liquid fermented organic manure (LFOM),
wherein the off-gas composition has a liquid to gas (L/G) ratio within the range between 15.3 to 15.6 liter/Nm³ and is sparged for a duration of 10-15 seconds.

2. The process as claimed in claim 1, wherein the LFM are added in a ratio of 0.1 ml per 10% lignin content and has CFU of 10¹¹ per ton of bio-digester slurry.

3. The process according to any one of claims 1 or 2, wherein the H₂S-oxidising microbes (SOD) are added in a ratio of 100 ml of SOD and has CFU of 10¹¹ per ton of bio-digester slurry.

4. The process according to any one of claims 1 to 3, wherein the off-gas composition is added at a flow rate of 10 m³/h for every 0.1% (w/w) increase in N content within the bio-digester slurry.

5. The process according to any one of claims 1 to 4, wherein the LFM is selected from the group consisting of *Pseudomonas putida MTCC 5869, Bacillus subtilis MTCC 5851, Bacillus sp. MTCC 5870, Pseudomonas putida MTCC 5871* or a combination thereof.

6. The process according to any one of claims 1 to 5, wherein the SOD is selected from the group consisting of *Thiobacillus sp. MTCC 25280, Thiobacillus sp. MTCC 25281, Lactobacillus sp. MTCC 25282, Mucor sp. MTCC 25283* or a combination thereof.

7. The process according to any one of claims 1 to 6, wherein the bio-digestor slurry is prepared from feedstocks selected from the group consisting of cattle dung, kitchen waste, organic fraction of municipal solid waste, paddy straw, and press mud.

8. The process according to any of claims 1 to 7, wherein the CO₂ content in the off-gas composition ranges from 80%-99%, and wherein the H₂S content in the off-gas composition ranges from 50-5000 parts per million (ppm), and wherein the off-gas composition has a moisture content of less than 5%.

9. The process according to any of claims 1 to 8, wherein the off-gas composition is sparged into the bio-digestor unit (3) of the bioreactor using micro-bubbler of a biogas purification unit (2).

10. The process according to any of claims 1 to 10, wherein the reactor is a column reactor.
